# EUROPEAN PATENT APPLICATION

(11) **EP 1 964 554 A1**
(43) Date of publication of application: **03.09.2008**
(21) Application number: 05825325.3
(22) Date of filing: 30.11.2005
(51) Int. Cl.: A61K 31/20, A23L 1/29

(54) **LIPID MIXTURE AND USE THEREOF FOR THE PREPARATION OF A PRODUCT THAT IS INTENDED FOR ENTERAL OR ORAL ADMINISTRATION**

(71) Applicant: Katry Inversiones, S.L., 6011 Badajoz (ES)
(72) Inventor: GIL HERNÁNDEZ, Angel, E-6011 Badajoz (ES); MARTÍNEZ DE VICTORIA MUÑOZ, Emilio, E-06011 Badajoz (ES); SAN ROMÁN PAIS, Paloma, E-06011 Badajoz (ES); RUIZ GUERRERO, Rosa, E-06011 Badajoz (ES)
(74) Representative: Gil-Vega, Victor
(86) International application number: PCT/ES2005/000656
(87) International publication number: WO 2007/063143

(57) **Abstract**

This invention relates to a novel mixture of lipids and the use thereof for preparing a food product intended for oral or enteral nutrition wherein the lipid mixture has a balanced and adequate fatty acid profile specially suited for using the same in complete nutritional products for oral or enteral administration. Such fatty acid profile, from vegetable and fish oils, provides a relatively low content of saturated fatty acids, a high content of medium-chain and monounsaturated fatty acids (oleic acid) and a balanced supply of essential (linoleic and α-linolenic) fatty acids, as well as long-chain polyunsaturated fatty acids of the Omega 3 series (n-3).

## Description

This invention relates to a novel lipid mixture and the use thereof for preparing a food product intended for oral or enteral nutrition.

Whether with oral or enteral administration products, there is a large number of social groups that require coverage of their nourishing needs with this type of nutritive products to avoid malnutrition. This malnourishment is more frequent in older population, since even when they do not suffer from any specific pathology associated to nutrition, this group presents factors closely related to the same, for example decreased number of papillae, lack of teeth that renders mastication difficult, less secretion of saliva and decreased metabolic rate and together with it, a decreased need to eat.

This type of artificial nutrition is also used in several pathological conditions, for example people affected by cerebrovascular accidents, multiple sclerosis, Parkinson's or Alzheimer's diseases, people suffering from mouth or throat cancer or esophageal damage, in general people showing hypermetabolic states with different etiologies (burns, traumas, surgery), chronic illnesses or people who have been subject to prolonged periods of reduced oral intake. All these possible situations have to be taken into account in the case of inpatients, where nutrition control is relatively easier, as well as in the case of outpatients where homecare is in charge of health staff or the patient's relatives or even himself/herself.

There are several formulations intended for oral or enteral nutrition, products made of a mixture of nutrients classified within the legal framework as "dietetic products for specific nutritional purposes". These may consist of nutritionally complete formulae, which contain an adequate quantity of each and all nutrients necessary for a balanced nutritional state, supplements, which are nutritionally incomplete and supplement an adequate oral diet, and finally modules, which contain one specific nutrient. In the case of oral administration formulae, additives such as flavours and aromas are added to improve palatability. Complete formula products, where the nourishing formula contains proteins, carbohydrates, fat, fibre and micro nutrients such as the applicant's T-Diet^{®} range of products are especially important. For example, US/4752618 describes a nutritional supplement containing fatty acids from fish and vegetables. US 5504072 describes enteral nutritional compositions containing polyunsaturated fatty acids with a lipid content between 4% and 30% based on the total caloric content of the composition. EP 0 994 657 B1 describes nutritional products containing a mixture of vegetal oils. However, in the light of the numerous nutritional studies carried out during the last years, it is evident that there is a need for including fatty acids from vegetables and fish in the diet so that about 30% to 35% of the dietary energy is provided by fats. The need of a balanced fat intake is most evident in the case of cardiovascular disease, being a diet with a non-appropriate fat content an important risk factor. Further to this fat balance, products intended for enteral or oral nutrition containing balanced mixtures of lipids must have good stability and meet the consumer demands as regards taste, flavour, texture, etc., especially if they are going to be ingested orally.

The novel lipid mixture of this invention, besides showing a balanced and adequate fatty acid profile is specially suited for its use in complete nutritional products for oral or enteral administration, since it facilitates a fatty acid profile with a relatively low content of saturated fatty acids, a high content of medium-chain and monounsaturated fatty acids (oleic acid) and a balanced supply of essential (linoleic and α-linolenic) fatty acids, as well as of long-chain polyunsaturated fatty acids such as those of the Omega 3 (n-3) series. This mixture has been used to develop optimal plasma and cell fatty acid profiles that limit the formation of oxidized compounds in blood plasma and at cell level, as well as the certain biologic factors involved in the metabolic syndrome.

In most of the diets, fats are a concentrated source of energy, they transport fat-soluble vitamins and provide essential fatty acids (at least 3-4%, especially linoleic acid); they can be found as medium-chain triglycerides with variable amounts of long-chain triglycerides. The first ones show a bioavailability as regards digestion, absorption and transportation higher than that of long-chain triglycerides; the second ones provide essential fatty acids. Even though there is competitiveness among them at the time of intestinal absorption, when mixtures of both types are administered, the total absorption of the same is increased as compared with the absorption shown when individually administered.

The lipid mixture of this invention consists in a mixture of several vegetal oils (98.4%) and purified fish oil (1.6%). The lipid profile provided by the lipid mixture of the invention is shown in the following Table:

**Table**

| **Lipid profile** | **g per 100 g of fat** |
|---|---|
| C 8:0 (caprylic acid) | 4.92 |
| C 10:0 (capryc acid) | 4.43 |
| C 12:0 (lauric acid) | 0.25 |
| C 14:0 (myristic acid) | 0.36 |
| C 15:0 (pentadecanoic acid) | 0.025 |
| C 16:0 (palmitic acid) | 12.07 |
| C 16:1 (palmitoleic acid) | 0.14 |
| C 17:0 (heptadecanoic acid) | 0.025 |
| C 18:0 (stearic acid) | 3.00 |
| C 18:1 (oleic acid) | 49.59 |
| C 18:2 (linoleic acid) | 19.95 |
| C 18:3 (linolenic acid) | 2.28 |
| C 20:0 (arachidic acid) | 0.43 |
| C 20:1 (c-11-eicosenoic acid) | 1.51 |
| EPA | 0.32 |
| C 22:0 (behenic acid) | 0.34 |
| DHA | 0.16 |
| C 24:0 (lignoceric acid) | 0.15 |

Medium-chain fatty acids (MCFA) Target: 10.3% Range (min-max): 8.5-10.3%. The relatively high amount of these MCFA in the formula provides a significant part of the total energy of the diet, easily absorbable without the aid of pancreatic lipase, while facilitating the obtainment of tissue energy since these fatty acids are oxidized in the mitochondrion without passage facilitated by the carnitine -dependant transport system.

The fatty acid profile present in this invention provides a monounsaturated:saturated fatty acid ratio of 2:1, being the presence of oleic acid (monounsaturated) majority and being saturated fatty acids mainly constituted by medium-chain fatty acids with very low values of lauric and myristic acids.

Lauric and myristic fatty acids: Target: 0.61 % Maximum range: 1.0% Proportion / MCFA: Range (min- max): 0-0.2%

Lauric and myristic acids are strongly atherogenic and the quantity of the same is limited to enhance the healthy cardiovascular property of the oil mixture present in the food product of the invention.

Likewise the proportion of stearic acid is quite lower than in other formulations of this type of preparations. This lipid mixture has a high bioavailability thanks to its relatively high content of short-chain fatty acids and it is not very atherogenic; in this regard there are several scientific evidences indicating that a high content of oleic acid decreases LDL cholesterol and increases HDL cholesterol, the presence of the most atherogenic acids known (C 12:0 and C 14:0) being limited.

| | |
|---|---|
| Saturated fatty acids >14 C: | Target: 15.8% Range (min- max): 12-21% |
| Total saturated fatty acids: | Target: 26.71% Range (min- max): 20-32% |
| Palmitic acid: Target: 12% | Range (min- max): 10-15% |
| Stearic acid: Target: 3% | Maximum 5% |
| Arachidic acid: Target: 0.43% | Maximum 0.5% |
| Behenic acid: Target: 0.34% | Maximum 0.5% |

The limit in the total amount of saturated fatty acids above 14C and in the total fatty acids is due to the importance of limiting the atherogenicity of the lipid mixture.

| | |
|---|---|
| Monounsaturated fatty acids (Oleic acid): | Target 49.59% Range (min-max): 45-55% |
| Proportion of total MCFA/SFA: Target: | 1.84% Range (min-max): 1.6-2.0 |

The high content of oleic acid in the formula tries to boost the healthy cardiovascular effects of the lipid mixture (they increase HDL cholesterol), as well as to favour its antioxidant effect.

Linoleic acid contribution is about 7% of the energy and α-linolenic acid contribution is 0.7%, being the ratio between these two acids of 1/10. This meets the most modern criteria (WHO report on fatty acids requirements) of supplementing fatty acids to cover the requirements of essentials fatty acids. Many mixtures currently in use for enteral or oral nutrition do not have adequate amounts of α-linolenic acids; in other formulations the amounts of linoleic acid are excessive and in many of them the ratio between these two acids is inadequate.

In the lipid mixture of the invention the MUFA:PUFA (poly-unsaturated fatty acids) ratio is about 2. This prevents an excessive unsaturation of cell membranes and limits their oxidation. On the other hand, it is well-known that an excessive intake of linoleic acid inhibits the formation of long-chain PUFA at intestinal and hepatic levels.

Total poly-unsaturated fatty acids (PUFA): Target 22.7% Range (min-max): 22-25%.

| | |
|---|---|
| Linoleic acid: | Target: 19.95% Range (min- max): 17.1-22.8% |
| α-Linolenic acid: | Target: 2.28% Range (min- max): 1.7- 2.4% |
| Eicosapentaenoic acid (EPA): | Target 0.32% Range (min-max): 0.3-0.34% |
| Docosahexaenoic acid (DHA): | Target 0.16% Range (min-max): 0.3-0.34% |
| Proportion MUFA/PUFA: | Target: 2.18 Range (min- max): 1.8-2.4 |
| Proportion PUFA/SFA: | Target: 0.85 Range (min- max): 0.75-1.00 |
| Proportion EPA/DHA: | Target: 2 Range (min- max): 1.8-2.2 |

The lipid mixture of the invention has been supplemented with adequate quantities of eicosapentaenoic acid (EPA) and docosahexaenoic acid (DHA) in a 2/1 ratio. The contribution of these long-chain PUFA of the n-3 series (Omega 3) limits the formation of triglycerides, which levels are high in metabolic syndrome, exerts a role as potent anti-inflammatory agent and contributes to limit platelet aggregation. In this sense, its use in certain populations such as older people is very interesting since they usually suffer cardiovascular conditions and other chronic inflammatory pathologies.

The mixture of oils is conveniently stabilized using a mixture of soy lecithin and α-tocopherol to limit the oxidation of its components and to limit the biologic oxidation of consumer subjects.

In one preferred embodiment of the lipid mixture of the invention, the amount of fat content in 100 mL of the product for enteral or oral nutrition is as follows:

| **Fats** | 4.19 g |
|---|---|
| From which | |
| Saturated fat, from which | 1.27 g |
| MCT | 0.46 g |
| monounsaturated | 2.52 g |
| Polyunsaturated, from which | 1.11 g |
| Linoleic acid | 0.98 g |
| Linolenic acid | 0.11 g |
| EPA | 0.013 g |
| DHA | 0.006 g |

## Claims

1. A lipid mixture and the use thereof in a food product intended for enteral or oral nutrition **characterized in that** such mixture of lipids contains purified vegetal and fish oils and **in that** it presents a lipid profile wherein the fatty acid proportions are the following: Medium-chain fatty acids (MCFA): 10.3%, [(min-max): 8.5-10.3%]; Lauric and myristic fatty acids: 0.61% [maximum 1.0%]; proportion of MCFA: 0-0.12%; saturated fatty acids >14C: 15.8%, [(min-max): 12-21%]; total saturated fatty acids: 26.71%, [(min-max): 20-32%]; total monounsaturated fatty acids: 49.59%, [(min-max): 45-55%]; proportion of total MUFA/SFA: 1.84%, [(min-max): 1.6-2.0]; Total poly-unsaturated fatty acids (PUFA): 22.7%, [(min-max): 22-25%]; proportion MUFA/PUFA: 2.18 (min-max): 1.8-2.4%]; proportion PUFA/SFA: 0.85 [(min-max): 0.75-1.00]; proportion EPA/DHA:2 [(min-max): 1.8-2-2].

2. The lipid mixture according to claim 1, **characterized in that** it contains a 98.4% of vegetal oils and 1.6% of fish oil.

3. The lipid mixture according to Claim 1, **characterized in that** the mixture of oils is conveniently stabilized by the use of a mixture of soy lecithin and α-tocopherol.

4. Food product intended for enteral or oral nutrition **characterized in that** it includes a lipid mixture according to Claim 1.
